# EUROPÄISCHE PATENTSCHRIFT

(11) **EP 2 152 913 B1**
(45) Veröffentlichungstag und Bekanntmachung des Hinweises auf die Patenterteilung: **18.03.2015**
(21) Anmeldenummer: 08749840.8
(22) Anmeldetag: 29.04.2008
(51) Int. Cl.: C12Q 1/68, G01N 33/569

(54) **DETEKTIONSVORRICHTUNG ZUR DETEKTION VON BIOLOGISCHEN MIKROPARTIKELN WIE BAKTERIEN, VIREN, SPOREN, POLLEN ODER BIOLOGISCHE TOXINE, SOWIE DETEKTIONSVERFAHREN**
DETECTION DEVICE FOR DETECTING BIOLOGICAL MICROPARTICLES SUCH AS BACTERIA, VIRUSES, SPORES, POLLEN OR BIOLOGICAL TOXINS, AND DETECTION METHOD
DISPOSITIF DE DÉTECTION PERMETTANT LA DÉTECTION DE MICROPARTICULES BIOLOGIQUES TELLES QUE DES BACTÉRIES, DES VIRUS, DES SPORES, DU POLLEN OU DES TOXINES BIOLOGIQUES, ET PROCÉDÉ DE DÉTECTION

(30) Priorität: 04.05.2007 DE 102007021387
(43) Veröffentlichungstag der Anmeldung: 17.02.2010
(73) Patentinhaber: Airbus Defence and Space GmbH, 85521 Ottobrunn (DE)
(72) Erfinder: FRIEDBERGER, Alois, 85667 Oberpframmern (DE); REIDT, Ulrich, 34613 Schwalmstadt (DE); MÜLLER, Gerhard, 85567 Grafing (DE)
(74) Vertreter: Flügel Preissner Kastel Schober
(86) Internationale Anmeldenummer: PCT/EP2008/055231
(87) Internationale Veröffentlichungsnummer: WO 2008/135452

(56) Entgegenhaltungen:
- EP-A- 1 445 331
- WO-A-00/65093
- WO-A-2004/009840

## Beschreibung

Die Erfindung betrifft eine Detektionsvorrichtung zur Detektion von biologischen Mikropartikeln wie z.B. lebende oder tote Bakterien, Viren, Sporen, Pollen oder biologische Toxine, die durch mittels Strahlung erfassbarer Sonden markierbar sind, insbesondere eine Detektionsvorrichtung zur Detektion von lebenden Bakterien, Viren oder biologischen Toxinen mittels fluoreszierender Nukleinsäure-Sonden oder Proteinen als Sonden. Die Erfindung betrifft außerdem ein Detektionsverfahren zum Detektieren von biologischen Mikropartikeln wie lebende Bakterien, Viren, Sporen, Pollen und/oder biologische Toxine in einem Fluid.

Bisherige Detektionsmethoden für lebende Bakterien benötigen einen zeitaufwändigen Kultivierungsschritt der Mikroorganismen. Diese herkömmliche Art der Lebenddetektion bzw. der Lebendzellzahlbestimmung ist enorm zeitaufwändig und kann nur in geeigneten biologischen Laborräumen (S1-Labor bis S4-Labor) durchgeführt werden. Eine alternative Methode zur Detektion von lebenden Bakterien ist die in-situ-Hybridisierung. Die in-situ-Hybridisierungs-Methode ist eine Standardtechnik, die bereits häufig in der Molekularbiologie angewendet wird. Zahlreiche Publikationen sind zu dieser Methode veröffentlicht worden.

In bezug auf Bakteriendetektion bietet die Firma Vermicon AG, München, fertige Detektionskits, d. h. Ensembles von Chemikalien, für unterschiedliche Bakterienstämme an.

Bisherige Detektionsverfahren einschließlich der in-situ-Hybridisierung sind in der WO 01/68900 A2, der WO 02/101089 A2, der DE 103 07 732 A1, der WO 2005/031004 A2, der US 6844157B2, der US 2005/064444 A1, der US/20050202 477 A1, der US 2005/0202476 A1 sowie der US 2005/0136446 A1 näher beschrieben. Es wird für weitere Einzelheiten ausdrücklich auf diese Dokumente aus dem Stand der Technik verwiesen.

Zurzeit ist die Analysemethode der in-situ-Hybridisierung in der Praxis jedoch nur mit einem sehr teuren und hochempfindlichen Fluoreszenzmikroskop möglich.

WO 2004/009840 A1 beschreibt eine Vorrichtung sowie ein Verfahren zur automatischen Detektion von biologischen Mikropartikeln, wobei Proben-, Reagenz- und Waschflüssigkeit über jeweils eine separate Pumpe sowie ein Mehrwege-Motorventil einem Filter zwecks Detektion bzw. Aufbereitung zugeleitet wird.

Die Erfindung hat sich zur Aufgabe gestellt, eine Vorrichtung und ein Verfahren zur Verfügung zu stellen, mit dem insbesondere lebende Bakterien, aber auch andere durch spezifische Sonden markierbare Mikropartikel und Mikroorganismen, wie insbesondere Viren oder biologische Toxine, schnell, sicher und unkompliziert automatisch detektierbar sind.

Diese Aufgabe wird durch eine Detektionsvorrichtung mit den Merkmalen des Patentanspruches 1 sowie ein Detektionsverfahren mit den Schritten des Patentanspruches 10 gelöst.

Vorteilhafte Ausgestaltungen der Erfindung sind Gegenstand der Unteransprüche. Eine vorteilhafte Verwendung ist Gegenstand des weiteren Nebenanspruches.

Vorteilhafte Ausgestaltungen der Erfindung ermöglichen insbesondere eine automatische Lebenddetektion von pathogenen Bakterien in Trinkwasser und Luft.

Insbesondere schafft die Erfindung ein Gerät zur schnellen, vorzugsweise automatischen Detektion von lebenden Bakterien ohne die Zuhilfenahme eines Fluoreszenzmikroskops.

Die Messergebnisse sind insbesondere dazu verwendbar, eine Korrelation zur LZZ (Lebendzellzahl; englisch CFU colony forming units) zu erreichen mit dem Ziel, eine Alternative zur zugelassenen Methode der zeitaufwändigen Kultivierung zu schaffen (welche den LZZ-Wert liefert).

Erfindungsgemäß ist ein Filter vorgesehen, das zum Ausfiltern der zu detektierenden Mikropartikel geeignet ist. Die Stoffe oder Chemikalien, die für unterschiedliche Markierungsschritte zum Markieren der Mikropartikel zwecks Detektion dienen, werden beispielsweise über ein Versorgungssystem bereitgestellt und ebenfalls zu dem Filter, insbesondere über das Filter, geleitet. Mittels eines Detektionssystems lässt sich die Strahlung zum Erfassen der Sonden erfassen und somit lassen sich die Mikropartikel erfassen. Das Filter und/oder das Fluidiksystem der Detektionsvorrichtung mittels Chemikalien regeneriert, gereinigt und/oder konditioniert wird.

Insbesondere für die Detektion von lebenden Bakterien wird beispielsweise ein mikromechanisches Filter eingesetzt. Das Filter weist ein mikromechanisches Filterelement und/oder eine Mikropartikel anziehende Membran, insbesondere eine Nitrocellulosemembran, auf. Es ist zum Vorsehen der Nitrocellulosemembran eine Nitrocellulosebahn vorgesehen, die zwischen Messungen weiterspulbar ist.

Ein Versorgungssystem liefert in einem solchen Fall insbesondere die für die einzelnen Schritte einer in-situ-Hybridisierung einzusetzenden Chemikalien, beispielsweise aus den zum Erfassen bestimmter Bakterien auf dem Markt erhältlichen Detektionskits.

Das Versorgungssystem kann vorzugsweise über Ventile und/oder Pumpen automatisch gesteuert werden, so dass eine vollautomatische Durchführung des Detektionsverfahrens ermöglicht ist. Das Versorgungssystem ist zur Lieferung mehrerer unterschiedlich markierter Sonden zur gleichzeitigen Detektion unterschiedlicher Mikropartikel ausgebildet.

Das Filter ist mehrmals verwendbar, indem es durch geeignete Chemikalien regeneriert und/oder konditioniert wird. Dies wird durch entsprechend gesteuerte Pumpen und Ventile ermöglicht.

Insbesondere kann das Versorgungssystem Teil eines Fluidiksystems sein, das nicht nur Chemikalien zum Markieren und/oder späteren Spülen und Reinigen der Vorrichtung liefert, sondern auch zu messende Fluide zu dem Filter liefern kann. Eine Quelle für das zu messende Fluid ist an das Ventilsystem angeschlossen, um es wahlweise zu dem Filter zu leiten. Die Messung der Strahlung ist jeweils an die verwendeten Sonden angepasst. Damit die Sonden Strahlung emittieren, können Fluoreszenzfarbstoffe verwendet werden, oder beispielsweise auch Raman-Labels, Quantum Dots, oder ähnliches. Falls mit einem Fluoreszenzfarbstoff markierte Sonden eingesetzt werden, wie dies aus der in-situ-Hybridisierung bekannt ist, wird vorzugsweise eine Lichtquelle zum Anregen des Fluoreszenzfarbstoffes, beispielsweise ein Laser, eingesetzt. Als Strahlungsmessgerät zum Messen von mit den Sonden korrelierter Strahlung wird vorzugsweise ein auf die bestimmte Fluoreszenzstrahlung sensitiver Lichtdetektor eingesetzt. Eine Auswerteeinheit ist zum Zählen von Strahlimpulsen oder der Ursprungspositionen ausgebildet und eingerichtet. Das Detektionssystem weist eine Lichtquelle zum Anregen von fluoreszierenden Sonden und einen Lichtdetektor zum Erfassen einer von angeregten fluoreszierenden Sonden ausgestrahlten Lichtstrahlung auf, wobei die Lichtquelle einen Laser oder eine LED aufweist. Der Lichtdetektor weist einen Photomultiplier und/oder ein zweidimensionales CCD-Array auf.

Der Lichtdetektor kann beispielsweise einen Photomultiplier aufweisen, um so die Intensität des Fluoreszenzlichtes festzustellen. Alternativ oder zusätzlich kann ein ortsauflösender Lichtdetektor, beispielsweise ein zweidimensional ortsauflösender Lichtdetektor eingesetzt werden. Ein solcher wird beispielsweise durch ein zweidimensionales CCD-Array gebildet. Ein solcher ortsauflösender Sensor kann Lichtimpulse sowie die Position ihres Ursprunges auf dem Filter feststellen. Aufgrund der größeren Auflösung sowie der einzelnen Sensorelemente lässt sich eine vorzugsweise softwaregesteuerte Auswerteeinheit anschließen, die z. B. die Lichtpunkte zählt und damit die Sonden und damit wiederum die Mikropartikel zählt. Mittels des Lichtdetektors wird die Lichtintensität gemessen, wobei für den Lichtdetektor ein Photomultiplier verwendet wird. Mittels eines ortsauflösenden Lichtdetektors werden Lichtstrahlung oder Lichtimpulse und die Position ihres Ursprungs auf dem Filter detektiert und die Lichtimpulse und/oder die Positionen mittels eines Datenverarbeitungssystems gezählt.

Zum Ausfiltern von lebenden Bakterien ist beispielsweise ein mikromechanisches Filterelement einsetzbar. Vorzugsweise werden mikromechanische Filter mit Porengrößen deutlich unterhalb der Größe der auszufilternden Mikropartikeln verwendet. Ein mikromechanisches Filter hat beispielsweise eine Porengröße von weniger als 0,8 µm, beispielsweise von etwa 0,45 µm, so dass Bakterien mit typischen Dimensionen von 1 µm sicher zurückhaltbar sind.

Andere interessante Mikropartikel, wie beispielsweise Viren oder biologische Toxine, haben teilweise geringere Abmessungen. Zum Ausfiltern dieser Mikropartikel lässt sich ein mikromechanisches Filter mit geringerer Porengröße oder ein anderes Filter, insbesondere ein Nitrocellulose-Filter, verwenden. Hierzu wird beispielsweise ein mikromechanisches Filter oder ein anderer poröser Körper als Auflage mit Nitrocellulose überschichtet. Die Nitrocellulosemembran kann beispielsweise durch eine Bahn bereitgestellt werden, die zum Austausch der wirksamen Nitrocellulosemembran bewegbar ist, so dass ein frisches Bahnstück als Filter für neue Messungen verwendbar ist.

Zum Nachweis insbesondere von Viren oder biologischen Toxinen können durch Strahlung erfassbare Proteine, zum Beispiel mit einem Fluoreszenzfarbstoff markierte Antikörper oder markierte Nukleinsäure-Sonden, verwendet werden.

Zur Erfassung von lebenden Bakterien werden vorzugsweise entsprechend markierte DNA-Sonden eingesetzt, die sich an die mRNA, welche nur von lebenden Bakterien mit ausreichender Lebensdauer bereitgestellt wird, anlagern. Durch Verwendung entsprechend unspezifischer DNA-Sonden, die zu Nukleinsäuren oder mRNA unterschiedlicher Bakterienarten passen, wird die Gesamtkonzentration von Bakterien mehrerer Bakterienarten erfasst, wobei durch die Verwendung einer Mischung von unterschiedlich markierten Sonden mehrere unterschiedliche Bakterienarten gleichzeitig detektiert werden.

Zusätzlich zu dieser bei der in-situ-Hybridisierung verwendeten SondenMarkierung kann auch ein PCR-Modul verwendet werden, um alternativ oder zusätzlich eine PCR-Detektionsmethode durchzuführen.

Bei der zum Beispiel auch in der Kriminalistik eingesetzten PCR-Methode werden DNA-Stücke multipliziert und anschließend detektiert.

Soll die Konzentration von Mikropartikeln in Flüssigkeiten festgestellt werden, beispielsweise die Anzahl von lebenden Bakterien im Trinkwasser, kann diese Flüssigkeit direkt in die Detektionsvorrichtung eingeleitet und über das Filter geleitet werden. Wenn eine Konzentration von Mikropartikeln in gasförmigen Medien, wie beispielsweise in der Luft, festgestellt werden soll, kann man diese Mikropartikel in einer vorgeschalteten Sammelvorrichtung, beispielsweise in einem Biosampler, zunächst in einer Flüssigkeit sammeln, und anschließend diese Flüssigkeit in das Detektionssystem übergeben, oder die Partikel aus der Luft direkt sammeln, indem die Luft durch den Filter geleitet wird.

Das Detektionsverfahren wird vollautomatisch gesteuert durchgeführt.

Vorteile der Erfindung und/oder von deren vorteilhaften Ausgestaltungen sind:
- Eine automatische, schnelle, unkomplizierte und sensitive Detektion von lebenden Bakterien ist mit der Detektionsvorrichtung an jedem beliebigen Ort möglich.
- Es kann auf aufwändige Kultivierungsschritte der Bakterien in einem biologischen Labor verzichtet werden.
- Eine mobile Detektion von lebenden Zellen ist möglich. Dadurch ist zum Beispiel eine schnelle Trinkwasseruntersuchung in einem Fahrzeug oder Flugzeug oder Raumfahrzeug oder in Verbindung mit einem Roboter, für zivile und/oder militärische Nutzung möglich.
- Die Vorrichtung und das Verfahren ermöglichen zum Beispiel eine schnelle Untersuchung von verdächtigen Flüssigkeiten zur Verhinderung eines biologischen Anschlags. Somit kann ein Beitrag zur öffentlichen Sicherheit geliefert werden.
- Als medizinische Nutzung ist z. B. eine schnelle Diagnose von bakteriellen Erkrankungen ohne eine aufwändige mikroskopische Untersuchung denkbar.

Ausführungsbeispiele der Erfindung werden nachfolgend anhand der beigefügten Zeichnungen näher erläutert: Darin zeigt:
Fig. 1 eine DNA-Sequenz zur Erläuterung der Grundlagen einer in-situ-Hybridisierung;
Fig. 2 eine schematische Darstellung des Prinzips der in-situ-Hybridisierung;
Fig. 3 eine schematische Darstellung einer schnellen Detektionsvorrichtung zur Erfassung lebender Bakterien in Trinkwasser;
Fig. 4 eine schematische Darstellung eines Ausführungsbeispiels einer schnellen Detektionsvorrichtung zur Erfassung lebender Bakterien in Fluiden mit einem Fluidiksystem mit Pumpen und Ventilen, sowie einer Detektionskammer mit Laser und Photomultiplier;
Fig. 5 eine schematische Zeichnung der Detektionskammer von oben mit einem mikromechanischem Filter;
Fig. 6 eine schematische Darstellung der Detektionskammer sowie eines Detektors einer weiteren Ausführungsform einer Detektionsvorrichtung;
Fig. 7 eine schematische Darstellung der Detektionskammer sowie von Detektoren einer weiteren Ausführungsform der Detektionsvorrichtung, mit welcher eine Kombination von in-situ-Hybridisierung mit einer PCR-Detektion durchführbar ist;
Fig. 8 eine schematische Darstellung der Detektionskammer nach einer weiteren Ausführungsform; und
Fig. 9 eine schematische Darstellung zur Erläuterung der Verwendung der Detektionsvorrichtung nach Fig. 8 zur Dektektion von Viren und biologischen Toxinen.

Im Folgenden werden Ausführungsbeispiele eines Verfahrens und eine Vorrichtung zur Detektion von Mikropartikeln, die durch mittels Strahlung erfassbare Sonden markierbar sind, anhand der Zeichnungen näher erläutert. Die Vorrichtung und das Verfahren sind insbesondere zur Detektion von lebenden Bakterien mittels Nukleinsäure-Sonden oder von Viren oder biologischen Toxinen mittels Proteinen einschließlich Antikörpern als Sonden geeignet. Bevor auf die Einzelheiten der Vorrichtung sowie das Verfahren eingegangen wird, werden zunächst die allgemeinen Verfahren zum Markieren von solchen Mikropartikeln mittels derartiger Sonden näher erläutert.

Im Trinkwasser befinden sich unterschiedliche lebende und tote Bakterien. Ein Teil davon kann pathogen sein. Relevant sind nur die lebenden Bakterien, da nur diese sich vermehren und zu einer Gesundheitsgefährdung führen können. Viele gebräuchliche Detektionsverfahren basierend auf Antikörpern oder PCR können jedoch nicht zwischen lebenden und toten Bakterien unterscheiden. Im Folgenden werden eine Vorrichtung und ein Verfahren vorgestellt, mit denen nur die tatsächlich lebenden - und dadurch relevanten - Bakterien erfasst werden können.

Bisherige Detektionsmethoden für lebende Bakterien benötigen einen zeitaufwändigen Kultivierungsschritt der Mikroorganismen. Die bakteriellen Proben werden hierzu auf speziellen Nährböden ausgestrichen und eine bestimmte Zeit bei bestimmter Temperatur inkubiert (bebrütet). Je nach Art der Bakterien kann dieser Kultivierungsschritt mehrere Tage dauern. Nach Beendigung der Inkubationszeit wird die "Colony forming Unit" (CFU), die Anzahl der entstandenen Bakterienkolonien, bestimmt. Diese herkömmliche Art der Lebenddetektion bzw. Lebendzellzahlbestimmung ist enorm zeitaufwändig und es besteht bisher keine Möglichkeit, lebende Bakterien schnell und unkompliziert zu detektieren.

Eine alternative Methode zur Detektion von lebenden Bakterien ist die in-situ-Hybridisierung. Gegenwärtig ist diese Analysemethode jedoch nur mit einem sehr teuren und hochempfindlichen Fluoreszenzmikroskop möglich. Mobile Feldversuche sind praktisch unmöglich, außerdem ist qualifiziertes Laborpersonal für die Bedienung erforderlich.

Zur Detektion von Mikroorganismen werden sehr oft weitere Detektionsmethoden herangezogen, wie zum Beispiel "Polymerase Chain Reaction" (PCR) oder "enzyme linked immuno sorbant assay" (ELISA). Diese Methoden erfassen aber ausschließlich die Anzahl der lebenden und toten Zellen, also die Gesamtzellzahl einer Probe. Eine Erfassung der Lebendzellzahl ist mit diesen Methoden nicht möglich.

### 2. Methoden zur Bakteriendetektion

### A) PCR

Die PCR-Methode (Polymerase Chain Reaktion) ist ein Verfahren, mit dem in einer Kettenreaktion kleinste Mengen eines DNA-Abschnitts vervielfältigt werden können (Amplifikation). Die PCR-Methode wird heute sehr oft eingesetzt, wenn anhand bestimmter DNA-Sequenzen Nachweise geführt werden sollen so etwa:
- in der Kriminalistik oder beim Vaterschaftstest,
- in der Mikrobiologie zur Detektion von lebenden und toten Bakterien (Gesamtzellzahl)
- in der medizinischen Diagnostik, wenn im Blut Viren-DNA nachzuweisen ist, oder
- in der Evolutionsbiologie, um Verwandtschaftsbeziehungen und Abstammungslinien zu verfolgen.

Um einen PCR-Nachweis führen zu können, müssen zwei kurze DNA-Stücke (Primer) vorhanden sein, die zu dem gesuchten DNA-Strang passen. Die von ihnen gestartete Kettenreaktion durchläuft bis zu 30 Zyklen, in denen die DNA-Menge jeweils verdoppelt wird. Aufgrund der Detektion über das Erbgut werden tote und lebende Bakterien nachgewiesen. Eine Detektion von ausschließlich lebenden Zellen ist mit der PCR-Methode nicht möglich.

### B) ELISA

ELISA (enzyme-linked, immuno sorbent assay) ist ein verbreitetes Verfahren, um einzelne Proteine (Antigen) nachweisen zu können. Dabei nutzt man die Mechanismen des Immunsystems: Wird eine Substanz vom Immunsystem als fremd erkannt, bildet es Antikörper, die an das fremde Molekül andocken und es so markieren. Diese so genannte Antikörper-Antigen-Interaktion wird für den ELISA-Test genutzt. Soll ein bestimmtes Protein nachgewiesen werden, müssen die dazu passenden Antikörper bekannt sein und zuvor mit verschiedenen gentechnischen oder zellbiologischen Verfahren hergestellt worden sein. Ist in einer Probe das gesuchte Protein vorhanden, binden es die auf ein Trägermedium aufgebrachten Antikörper. Nach der Antigen-Antikörper-Interaktion wird eine von Enzymen gesteuerte Reaktion ausgelöst, die zu einem sichtbaren Farbniederschlag führt. ELISA-Tests sind heute in der medizinischen Diagnostik weit verbreitet. Sie werden aber auch in vielen anderen Bereichen genutzt, wenn einzelne Proteine nachzuweisen sind. Im Falle einer Bakteriendetektion werden die bakterienspezifischen Oberflächenproteine von dem Antikörper erkannt. Es werden sowohl lebende als auch tote Bakterien erkannt (Gesamtzellzahl).

### C) In-situ-Hybridisierung

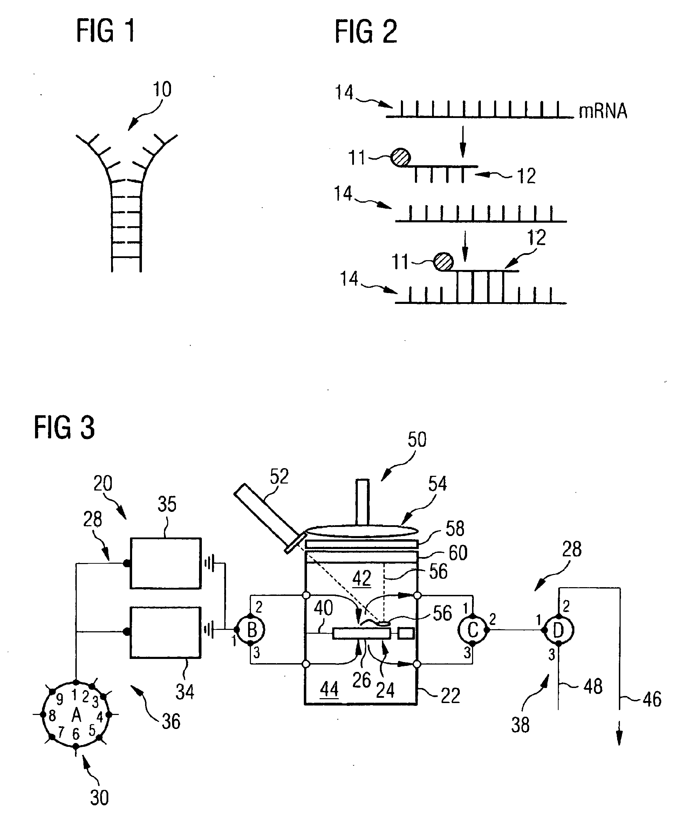

Im Folgenden wird auf Fig. 1 Bezug genommen. Eine DNA-Sequenz 10 kann man sich als einen "Reißverschluss" vorstellen. Die "Zähne" dieses Reißverschlusses sind die Basen Adenin (A), Cytosin (C), Guanin (G) und Thymin (T). Die Information, die die DNA enthält, ist verschlüsselt in der Reihenfolge dieser vier Buchstaben entlang des "Reißverschlusses". Gegenüberliegend "Zähne" bilden dabei immer nur entweder AT- oder GC-Paare. Die Sequenz ACGCT etwa hat als komplementäres Gegenüber die Buchstabenfolge TGCGA.

Bei der Hybridisierung wird der "Reißverschluss" durch Erhitzen geöffnet, so dass Einzelstränge vorliegen. Kurze, ebenfalls einzelsträngige DNA-Stücke, die als Sonden verwendet werden, sollen nun ihr passendes Gegenstück auf dem langen Einzelstrang finden. An dieser Stelle schließt sich der Reißverschluss beim Abkühlen wieder.

Man kann dies anhand von Markierungen an der Sonde (z. B. durch einen Fluoreszenzfarbstoff 11, siehe Fig. 2) sichtbar machen. Auf diese Weise kann herausgefunden werden, ob spezifische Sequenzen, die z. B. für bestimmte Marker-Gene stehen, in der untersuchten DNA vorhanden sind oder nicht (s. Punkt A: PCR).

Im Fall der in-situ-Hybridisierung, die im Folgenden näher anhand Fig. 2 erläutert wird, binden die fluoreszenzmarkierten Sonden 12 nicht an DNA, sondern an die mRNA 14 an, die sogenannte "Abschrift" der DNA. Die mRNA14 ist eine Art Kopie der DNA. Aus der in der mRNA transkribierten Information werden Proteine erzeugt (Proteinbiosynthese).

In toten Bakterien hat diese mRNA 14 eine sehr kurze Lebensdauer (Mikrosekunden) und wird sofort nach dem Ableben der Bakterien abgebaut.

Mit der in-situ-Hybridisierungs-Methode wird die mRNA der lebenden Zellen konserviert (Fachausdruck: "fixiert") und dann detektiert. D. h. es werden ausschließlich die lebenden Bakterien detektiert (Lebendzellzahl), da tote Bakterien keine mRNA mehr enthalten.

Da bei diesem Verfahren DNA an (m)RNA bindet, spricht man von Hybridisierung.

Auch eine Kombinatin von in-situ-Hybridisierung und PCR ist möglich. Hierbei wird die ebenfalls fixierte DNA der Bakterien zu einer Amplifikation herangezogen. Zum Beispiel wird diese als in-situ-PCR bezeichnete Methode für einen Parallelnachweis von Viren in der zu untersuchenden Probe verwendet.

Die in-situ-Hybridisierungs-Methode hat sich mittlerweile zu einer Standardtechnik entwickelt, die bereits häufig in der Molekularbiologie angewendet wird. Für weitere Einzelheiten zu den einzelnen Schritten der in-situ-Hybridisierung sowie der übrigen hier vorgestellten Detektionsverfahren wird ausdrücklich auf die WO 01/68 900, WO 02/101089 A2, DE 103 07 732 A1, WO 2005/031004, US 6 844 157 A1, US 2005/064444 A, US 2005/0202477 A1, US 2005/202476 A1, US 2005/0136446 A1 verwiesen.

Es sind auch bereits fertige Detektionskits mit Ensembles von Chemikalien auf dem Markt erhältlich, beispielsweise von der Fa. Vermicon AG, Emmy-Noether-Strasse 2, D-80992 München, Deutschland. Allerdings bedürfen die derzeit auf dem Markt erhältlichen Detektionskits eines Fluoreszenzmikroskops. Zudem müssen die Chemikalien fachgerecht angewendet werden. Dafür bedarf es Fachpersonal.

### Ausführungsbeispiele:

In Fig. 3 ist hingegen eine Detektionsvorrichtung 20 dargestellt, mit der eine vollautomatische Detektion lebender Bakterien ohne Verwendung eines Fluoreszenzmikroskops durchführbar ist. Die Detektionsvorrichtung 20 weist eine Detektionskammer 22 auf, in der ein Filter 24 untergebracht ist, das zum Ausfiltern der zu detektierenden Mikropartikeln oder Mikroorganismen aus dem zu untersuchenden Fluid geeignet ist.

Zur Detektion von lebenden Bakterien ist das Filter 24 als mikromechanisches Filter aufgebaut und weist hierfür ein mikromechanisches Filterelement 26 auf. Das mikromechanische Filter und dessen Filterelement 26 haben beispielsweise einen Aufbau, wie er näher in der nicht vorveröffentlichten deutschen Patentanmeldung DE 10 2006 026 559.9 beschrieben und gezeigt ist. Es wird für weitere Einzelheiten ausdrücklich auf die DE 10 2006 026 559 verwiesen. Der Inhalt dieser deutschen Patentanmeldung gehört durch Inbezugnahme auch zum Offenbarungsgehalt der hier vorliegenden Patentanmeldung.

Die Detektionsvorrichtung 20 weist weiter ein Fluidiksystem 28 auf, mit dem das zu untersuchende Fluid, beispielsweise Trinkwasser 30, welches auf das Vorhandensein von lebenden Bakterien 32 untersucht werden soll, zum und durch das Filter 24 geleitet wird.

Das Fluidiksystem 28 hat eine oder mehrere Pumpen 34, 35 sowie mehrere Motorventile A, B, C, D. Die Pumpen 34, 35 und die Ventile A, B, C, D sind durch eine nicht näher dargestellte Steuerung, beispielsweise ein angeschlossenes Computersystem mit entsprechender Software, automatisch steuerbar. Ein Teil des Fluidiksystems 28 dient außerdem als Versorgungssystem 36, mit dem Chemikalien zur Markierung der Bakterien 32 oder der sonstigen zu detektierenden Mikroorganismen oder Mikropartikel mit durch Strahlung erfassbarer Sonden, beispielsweise die fluoreszenzmarkierten Sonden 12, zu dem Filter 24 geleitet werden können. Ein weiterer Teil des Fluidiksystems 28 dient als Entsorgungssystem oder Abflusssystem 38 zum Abführen der Stoffe und Proben aus der Detektionskammer 22.

Das Versorgungssystem 36 des Fluidiksystem 28 weist genauer ein Motorventil A mit einem Ausgang A1 und mehreren Eingängen A2 bis A9 auf. Die Tabelle 1 zeigt die Belegung der Eingänge für ein Anwendungsbeispiel

**Tabelle 1: Belegung der Eingänge des Motorventils A in einem Anwendungsbeispiel**

| Ventileingang | A2 | A3 | A4 | A5 | A6 | A7 | A8 | A9 |
|---|---|---|---|---|---|---|---|---|
| | 20 % EtoH | H₂O | Reserve (Eingang ist frei) | PBS | Trinkwasserprobe | Antikörper | "wash buffer" (Waschpuffer) | 0,5 M NaOH |

An den Ausgang A1 schließen sich zwei parallel geschaltete Pumpen 34 und 35 mit unterschiedlichen Pumpleistungen an. Eine erste Pumpe 34 arbeitet beispielsweise in einem Arbeitsbereich von 6 bis 70 ml/min und eine zweite Pumpe 35 arbeitet in einem Bereich von 0,1 bis 7 ml/min. Je nach Steuerung und Schaltung der Pumpen 34, 35 lassen sich genau dosierte Mengen von aus dem Motorventil A gelieferten Fluiden in Richtung Detektionskammer 22 pumpen.

Auf die Pumpen 34, 35 folgt ein 3/2-Wege-Ventil B, mit dem der Ausgang der Pumpen 34, 35 wahlweise auf eine der beiden Seiten des Filters 24 geleitet werden kann. Die Detektionskammer 22 ist hierzu durch eine Trennung 40 in zwei Teilkammern 42 und 44 geteilt, die über das Filter 24 miteinander verbunden sind. Während der Eingang B1 des Ventils B mit dem Ausgang der Pumpen 34, 35 verbunden ist, ist ein Ausgang B2 des Ventils B mit der ersten Teilkammer 42 und ein zweiter Ausgang B3 des Ventils B mit der zweiten Teilkammer 44 verbunden. Je nach Beschickung der ersten oder zweiten Teilkammer 42 bzw. 44 kann so das Filter 24 durch entsprechende Flüssigkeiten belegt, gereinigt und/oder konditioniert werden. Entsprechende Strömungen können auch über das Abfluss-System 38 erreicht werden, wo ein erster Eingang C1 eines 3/2-Wege-Ventils C mit der ersten Teilkammer 42 und ein zweiter Eingang C3 mit der zweiten Teilkammer 44 verbunden ist. Mit dem 3/2-Wege-Ventil C kann wahlweise der erste Eingang C1 oder der zweite Eingang C3 auf einen Ausgang C2 geschaltet. Der Ausgang C2 ist wiederum mit einem Eingang D1 eines weiteren 3/2-Wege Ventils D verbunden. Dieser Eingang D1 kann durch das Ventil D wahlweise auf einen Abfluss 46 zur Entsorgung oder auf einen Sammelanschluss 48 zum Sammeln von Antikörpern geschaltet werden.

Das heißt, durch die Anordnung von Öffnungen zur Flüssigkeitszufuhr auf beiden Seiten des Filters 24 und Öffnungen zur Abfuhr der Flüssigkeit auf beiden Seiten des Filters 24, sowie durch die Ventile B und C wird erreicht, dass der Filter 24 in beiden Richtungen durchströmbar ist. Durch das umschaltbare Ventil A, das an zwei oder mehr Flüssigkeitsbehälter angeschlossen ist, kann der Filter 24 mit Chemikalien zum Markieren, Reinigen oder Konditionieren durchströmt werden.

Zur Detektion der mit den Hybridisierungs-Sonden oder fluoreszenzmarkierten Proteinen bzw. Antikörpern markierten Bakterien 32 ist weiter ein Strahlenmessgerät 50 an der Detektionskammer 22 angeschlossen.

Um z. B. Bakterien 32, die mittels fluoreszenzmarkierten Sonden 12 markiert sind, zu detektieren, ist eine Lichtquelle in Form eines Lasers 52, der zum Anregen des Fluoreszenzfarbstoffes 11 geeignete Strahlung, beispielsweise Licht mit der Wellenlänge 405nm, aussendet, vorgesehen. Diese Einschränkung der Wellenlänge lässt sich weiter verbessern durch ein der Lichtquelle nachgeschaltetes optisches Filter.

Das Strahlenmessgerät 50 weist in dem dargestellten Ausführungsbeispiel einen Lichtdetektor 54 auf, um die hier verwendete Strahlung, nämlich Fluoreszenzlicht 56 von den Bakterien 32 zu detektieren. Zur genaueren Erfassung solcher Strahlung weist das Strahlenmessgerät 50 weiter ein optisches Filterelement 58 auf, das nur die zu messende Strahlung, also hier das zu messende Fluoreszenzlicht 56, beispielsweise Fluoreszenzlicht bei einer Wellenlänge von 455nm, durchlässt.

Zum Anschluss des Strahlenmessgeräts 50 ist die Detektionskammer 22 an einer Seite mit einem Quarzglas 60 verschlossen. Durch das Quarzglas 60 hindurch erfolgt die Anregung mittels des Lasers 52 sowie die Lichtdetektion mittels des Lichtdetektors 54.

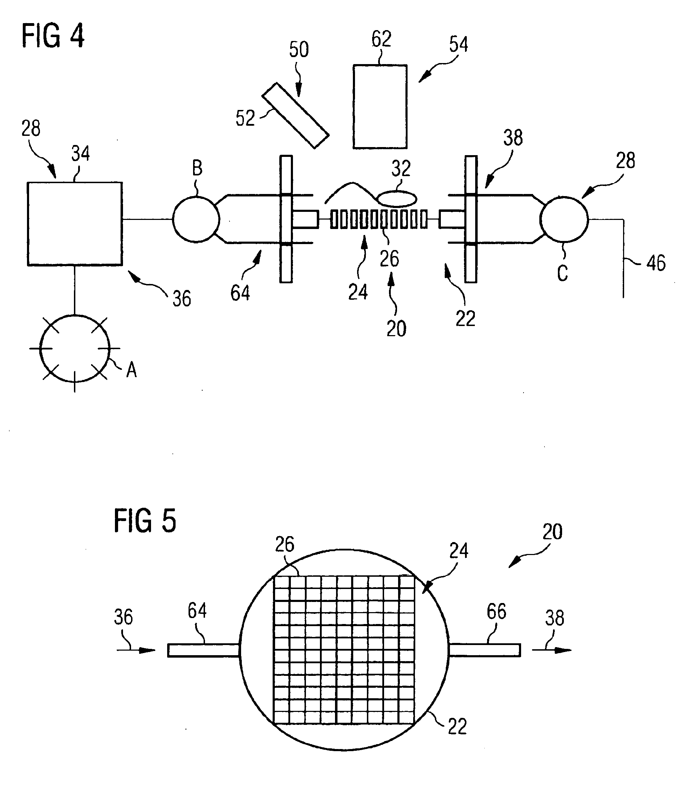

In Fig. 4 ist eine Übersichtszeichnung über eine speziellere erste Ausführungsform der Detektionsvorrichtung 20 dargestellt, wobei die Detektionskammer 22 sowie das Fluidiksystem 28 lediglich schematisch angedeutet ist. In dem in Fig. 4 dargestellten Ausführungsbeispiel weist der Lichtdetektor 54 ein Gerät zur Messung der Lichtintensität, hier in Form eines Photomultipliers 62 auf. Es kommt hier ein vereinfachtes Fluidiksystem 28 mit nur einer Pumpe 34 und einem vereinfachten Abfluss-System 38 zum Einsatz.

Mit der in Fig. 3 und 4 dargestellten Detektionsvorrichtung 20 lässt sich eine vollautomatische Detektion von lebenden Mikroorganismen mit der Methode der in-situ-Hybridisierung durchführen. In diesem System werden nun erstmals lebende Bakterien 32 mit der in-situ-Hybridisierung-Methode schnell und sicher detektiert. Herzstück des Gerätes bzw. der Detektionsvorrichtung 20 ist die Detektionskammer 22, die das mikromechanische Filterelement 26 enthält. Durch das an die Detektionskammer 22 angeschlossene Fluidiksystem 28 (Pumpen 34, 35, Leitungen oder Verschlauchungen sowie Ventile A, B, C, D) wird die zu untersuchende Probe (zum Beispiel Wasser 30 mit Bakterien 32) über das Filterelement 26 gepumpt.

Da das mikromechanische Filterelement 26 eine Porengröße von zum Beispiel 0,45 µm aufweist und Bakterien 32 einen Durchmesser von z. B. ca. 1 µm besitzen, reichern sich die Bakterien 32 auf der Filteroberfläche an.

Nach der Anreicherung werden die Bakterien 32 für die eigentliche Detektion präpariert. Hierzu werden mit Hilfe des Fluidiksystems 28, insbesondere mit Hilfe des Versorgungssystems 36, die für in-situ-Hybridisierung notwendigen Chemikalien über das Filterelement 26 gepumpt.

Die eigentliche Detektion erfolgt mit einer Lichtquelle, also zum Beispiel dem Laser 52, die den Fluoreszenzfarbstoff 11, mit dem die DNA-Sonde 12 markiert wurde, anregt. Das durch den angeregten Fluoreszenzfarbstoff 11 emittierte Fluoreszenzlicht 56 wird im Anschluss mit dem Photomultiplier 62 (PMT) detektiert. Spezielle optische Filter - hier das optische Filterelement 58 - sorgen dafür, dass ausschließlich von den markierten Bakterien 32 abgegebenes Fluoreszenzlicht 56 in den Photomultiplier gelangt.

Fig. 5 zeigt eine schematische Darstellung der Detektionskammer 22 mit dem Filter 24 von oben. Das zu erkennende Gitter stellt das mikromechanische Filterelement 26 dar. Außerdem sind Anschlüsse 64 und 66 an das Versorgungssystem 36 bzw. das Abfluss-System 38 des Fluidiksystems 28 dargestellt.

Zielorganismen für eine Lebenddetektion sind z. B. pathogene Keime. Aufgrund der Basensequenz der fluoreszenzmarkierten Sonde 12 ist es möglich, nicht nur ganz spezifisch eine pathogene Bakterienart (z. B. *E. coli*) zu detektieren, sondern es ist auch möglich, Bakterien auf einer höheren taxonomischen Ebene zu erfassen. So wäre es im Falle *E. Coli* möglich, dass alle Coli-Formen in einer Probe detektiert werden könnten.

Indem die Basissequenz der DNA-Sonde 12 unspezifischer gestaltet wird, kann es möglich werden, auf noch höherer taxonomischer Ebene, z. B. viele Enterobakterien, zu detektieren.

Auch ein gleichzeitiges Detektieren von unterschiedlichen Bakterienarten ist möglich. Hierzu kann man eine Mischung von unterschiedlich markierten Sonden 12 einsetzen, wobei für die jeweilige Bakterienart ein bestimmter Fluoreszenzfarbstoff 11 verwendet werden kann. Dadurch lässt sich bestimmen, welche Bakterien 32 in welcher Konzentration vorhanden sind, wenn mit unterschiedlichen Wellenlängen angeregt wird.

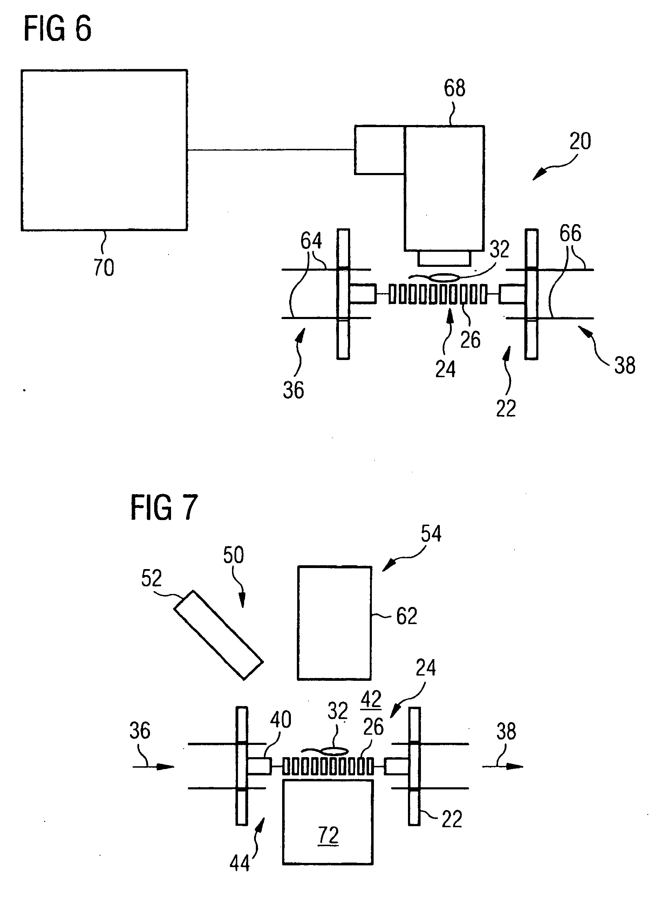

In Fig. 6 ist die Detektionskammer 22 mit dem Filter 24 sowie der Lichtdetektor 54 einer weiteren Ausführungsform der Detektionsvorrichtung 20 schematisch dargestellt. Bei dieser Ausführungsform gemäß Fig. 6 weist der Lichtdetektor 54 keinen Photomultiplier 62, sondern einen ortsauflösenden Detektor, nämlich hier genauer einen zweidimensionalen ortsauflösenden Detektor in Form eines zweidimensionalen CCD-Arrays 68 auf, das an eine Auswerteeinheit, beispielsweise ein Computersystem 70, angeschlossen ist.

Erfolgt die Detektion nicht über einen Photomultiplier 62, sondern mit einem zweidimensionalen ortsauflösenden Detektor, wie zum Beispiel dem CCD-Array 68, flächig über dem Filter 24, so lässt sich die Position der einzelnen Bakterien 32 auf dem Filter 24 bestimmen. Mittels einer Optik bestehend aus optischen Linsen lässt sich die Darstellung bzw. Auflösung auf dem CCD-Array 68 verbessern.

Mit einer geeigneten Software lassen sich dadurch die einzelnen Bakterien 32 zählen. Dadurch kann sich die tatsächliche Anzahl der Bakterien 32 genauer bestimmen lassen als bei einer Messung mit Photomultiplier 62. Bei letzterem wird nämlich nur die gesamte Lichtintensität gemessen. Aus der durch den Photomultiplier 62 erhaltenen Lichtintensität wird über eine geeignete Eichung abgeleitet, wie viele Bakterien 32 vorhanden waren. Das in Fig. 6 dargestellte Detektionssystem kann dagegen gleich eine Anzahl der Bakterien 32 ausgeben.

Die Ungenauigkeit einer Messung über die Lichtintensität ist umso größer, je unterschiedlicher die Konzentration von mRNA 14 in unterschiedlichen Bakterien 32 ist.

Um mit den zuvor beschriebenen Ausführungsbeispielen der Detektionsvorrichtung 20 Bakterien 32 in Luft oder sonstigen gasförmigen Medien zu detektieren, können diese, zum Beispiel automatisch, über eine geeignete Sammelvorrichtung in einer Flüssigkeit gesammelt werden und mit der Flüssigkeit, beispielsweise über den Anschluss A6 in die Detektionsvorrichtung 20 überführt werden. Geeignete Sammelvorrichtungen sind zum Beispiel unter der Bezeichnung Biosampler von der Fa. SKC Inc., Eighty Four, PA, USA auf dem Markt erhältlich.

Alternativ lässt sich Luft direkt durch den Filter leiten, um darauf die Partikel anzureichern.

Das mit der Detektionsvorrichtung 20 durchführbare Detektionsverfahren kann die heute zertifizierten Kultivierungsmethoden zur LZZ-Bestimmung (Lebendzellzahl) ersetzen. Hierzu werden die verwendeten Sonden 12 so ausgewählt, dass die damit detektierten Bakterien 32 ähnlich repräsentativ sind, wie die durch die konventionellen LZZ-Bestimmung detektierten Bakterien.

In Fig. 7 ist noch die Detektionskammer 22 mit dem Filter 24 sowie dem Strahlungsmessgerät 50 einer weiteren Ausführungsform der Detektionsvorrichtung 20 schematisch dargestellt.

Die Ausführungsform gemäß Fig. 7 weist, hier in der zweiten Teilkammer 44, ein Zusatzmodul 72 zur PCR-Detektion von DNA-Sequenzen auf. Das Zusatzmodul 72 ist beispielsweise eine Vorrichtung zum Durchführen einer real-time PCR. Solche Vorrichtungen sind auf dem Markt erhältlich, beispielsweise von der Fa. Fluidikm Corp., South San Francisco, USA.

Durch das Zusatzmodul 72, das zusätzlich zu der eigentlichen Detektionsvorrichtung 20 eingesetzt wird, ist eine Kombination von in-situ-Hybridisierung und PCR möglich. Beispielsweise kann eine sogenannte in-situ-PCR für eine nachträgliche Detektion von Viren verwendet werden. Die eigentliche Detektion könnte mit dem Verfahren der real-time-PCR ablaufen. Dabei werden während der Amplifikation Reporter-Moleküle freigesetzt, die aufgrund ihrer Fluoreszenz mit einem Photomultiplier detektiert werden können.

Hierzu ist das Zusatzmodul 72 mit einem eigenen Strahlenmessgerät zur Fluoreszenzlichtmessung ausgerüstet, oder es nutzt das entsprechend ausgebildete Strahlenmessgerät 50 der Detektionsvorrichtung 20.

Mit der Ausführungsform von Fig. 7 kann insbesondere die im Folgenden näher dargestellte besondere Ausführungsform des Detektionsverfahrens durchgeführt werden. Es wird eine Kombination von in-situ-Hybridisierung mit PCR durchgeführt. Bei der in-situ-Hybridisierung werden nach dem Fixieren der Bakterien 32 DNA-Sonden 12 zugegeben. Die nicht gebundenen DNA-Sonden 12 werden anschließend weggespült. Werden nun die gebundenen DNA-Sonden 12 wieder von der mRNA 14 gelöst und durch PCR amplifiziert, so lässt sich dadurch eine Lebendbakteriendetektion durchführen, wodurch sogar extrem kleine Bakterienkonzentrationen nachgewiesen werden können.

Die PCR-Methode dient hierbei also nicht zur Bestimmung der Gesamtzahl (toter und lebender Bakterien); es werden vielmehr die zuvor an die mRNA 14, die nur in Lebendbakterien mit ausreichender Lebensdauer vorhanden ist, angebundenen DNA-Sonden detektiert, die durch die PCR um ein Vielfaches multipliziert werden.

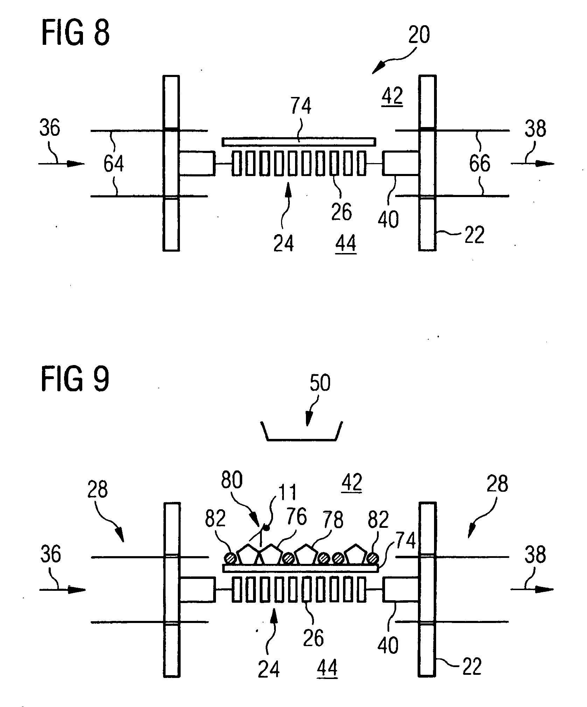

In Fig. 8 ist noch die Detektionskammer 22 sowie das Filter 24 einer weiteren Ausführungsform der Detektionsvorrichtung 20 dargestellt, mit der auch Viren und biologische Toxine automatisch detektierbar sind. Hierzu ist eine Membran, insbesondere eine Nitrocellulosemembran 74 vorgesehen. Die Nitrocelluloseschicht 74 kann beispielsweise auf irgendeinem durchlässigen Körper als Auflage vorhanden sein. In der in Fig. 8 und Fig. 9 dargestellten Ausführungsform der Detektionsvorrichtung 20 ist die Nitrocellulosemembran 74 als Überschichtung des mikromechanischen Filterelements 26 vorgesehen.

Fig. 9 zeigt das Prinzip der Filterung von Viren 76 und biologischen Toxinen 78 mittels der Nitrocellulose sowie die Detektion mittels fluoreszenzmarkierter Antikörper als Sonden, die bei diesem Detektionsverfahren zum Nachweis eingesetzt werden.

Fig. 8 und 9 verdeutlichen somit eine weitere Anwendung der schnellen Detektionsvorrichtung 20 zur Detektion von pathogenen Viren 76 und biologischen Toxinen 78.

Viren 76 sind kleine Partikel (25 - 500nm), die aus einer Proteinhülle und je nach Virusart einem RNA- oder DNA-Genom bestehen. Sie besitzen keinen eigenen Stoffwechsel, sondern vermehren sich ausschließlich durch lebende Zellen. Bekannte Beispiele sind Influenzaviren, Ebolaviren oder Pockenviren.

Biologische Toxine 78 sind äußerst stabile Proteine, die meistens von Bakterien, Einzellern oder Pflanzen als Stoffwechselprodukte gebildet werden. Oft werden diese Toxine in das umgebende Medium abgegeben und reichern sich dort in hohen Konzentrationen an. Im militärischen Bereich können biologische Toxine als biologische Waffen eingesetzt werden.

Bekannte Vertreter biologischer Toxine sind das Botulinumtoxin (Botox) und das Rizin, das aus den Kernen der Rizinuspflanze gewonnen werden kann.

Aufgrund der geringen Größe können Viren 76 und biologische Toxine 78 nur eingeschränkt auf mikromechanischen Filtern angereichert werden. Durch eine Überschichtung des mikromechanischen Filterelementes 26 oder eines anderen durchlässigen Körpers, der als Auflage dient, mit einem geeigneten absorbierenden Material, insbesondere Nitrocellulose 74, ist es dennoch möglich, Viren 76 und biologische Toxine 78 erfolgreich zu filtrieren.

Nitrocellulose besitzt eine sehr hohe Affinität gegenüber Proteinen und Nukleinsäuren. In Bezug auf seine Oberflächenbeschaffenheit werden Proteine irreversibel an die Nitrocellulosemembran 74 gebunden. So können ganze Viren mit ihren Oberflächenproteinen fest an die Nitrocellulosemembran 74 gebunden werden.

Das Binden von Proteinen und ganzen Viren auf Nitrocellulose ist in der biologischen Forschung eine verbreitete Methode und wird in zahlreichen Publikationen beschrieben ("Westernblot" oder "Dotblot").

Nachdem die Viren 76 an der Membran 74 haften, können die noch freien Stellen der Membran mit bestimmten Proteinen (z. B. BSA Bovines Serum Albumin) 82 aufgefüllt werden. Dieser Vorgang wird als Blocken der Membran bezeichnet. Nach dem Blocken erfolgt die eigentliche Detektion mit spezifischen Antikörpern 80, die mit einem Fluoreszenzfarbstoff 11 markiert wurden.

Durch die besondere Eigenschaft der Nitrocellulose ist es möglich, die Probe und sämtliche Flüssigkeiten, die für die Detektion notwendig sind, durch die Membran 74 zu saugen. Sollte das Saugen durch die Membran 74 durch irgendwelche Gründe eingeschränkt oder nicht möglich sein, so wäre auch ein oberflächliches Abspülen der Membran 74 mit dem Detektionsgerät möglich. Hierzu wäre nur eine andere Schaltfolge der 3/2-Wege-Ventile B, C notwendig, die sich links und rechts neben der Detektionskammer 22 befinden (Fig. 4).

Alle Schritte aller oben beschriebenen Detektionsverfahren können mit dem hier entwickelten Gerät - Detektionsvorrichtung 20 - vollautomatisch ablaufen, so dass Analysen auch ohne speziell ausgebildetes Fachpersonal möglich sind.

Neben der Untersuchung von Flüssigkeiten könnte ein mit Nitrocellulose ausgerüstetes Gerät auch für Luftuntersuchungen eingesetzt werden. Soll nun die Detektion der in der Luft befindlichen Viren 76 oder Toxine 78 erfolgen, so kann das gleiche automatische Programm wie für die Flüssigkeiten angewendet werden, wobei die Luft oder ein anderes gasförmiges Medium durch das Filter gepumpt wird.

Um zahlreiche Messungen hintereinander durchzuführen, lässt sich zum Verfügungsstellen der Membran 74 eine Endlosrolle aus Nitrocellulose verwenden, die z. B. nach jeder Messung eine Einheit weitergespult wird, wodurch das Filter 24 vor jeder neuen Messung frisch ist.

### Bezugszeichenliste

- 10: DNA-Sequenz
- 11: Fluoreszenzfarbstoff
- 12: fluoreszenzmarkierte Sonde
- 14: mRNA
- 20: Detektionsvorrichtung
- 22: Detektionskammer
- 24: Filter
- 26: mikromechanisches Filterelement
- 28: Fluidiksystem
- 30: Trinkwasser
- 32: Bakterien
- 34: Pumpe
- 35: Pumpe
- 36: Versorgungssystem
- 38: Abfluss-System
- 40: Trennung
- 42: erste Teilkammer
- 44: zweite Teilkammer
- 46: Abfluss
- 48: Sammelanschluss
- 50: Strahlenmessgerät
- 52: Laser
- 54: Lichtdetektor
- 56: Fluoreszenzlicht
- 58: optisches Filterelement
- 60: Quarzglas
- 62: Photomultiplier
- 64: Zulauf
- 66: Ablauf
- 68: CCD-Array
- 70: Computersystem (Auswerteeinheit)
- 72: Zusatzmodul
- 74: Membran, insbesondere Nitrocellulose
- 76: Viren
- 78: biologische Toxine
- 80: fluoreszenzmarkierte Proteine
- 82: Proteine, insbesondere BSA
- A: Ventil
- B: Ventil
- C: Ventil
- D: Ventil

## Patentansprüche

1. Detektionsvorrichtung (20) zur Detektion von biologischen Mikropartikeln (32, 76, 78), die durch Sonden (12, 80) markierbar sind, die mittels Strahlung (56) erfassbar sind, wobei Strahlung emittierende Nukleinsäure-Sonden (12) oder Strahlung emittierende Proteine als Sonden (80) verwendet werden, mit:
einem zum Ausfiltern der zu detektierenden Mikropartikel (32, 76,78) aus einem zu messenden Fluid (30) geeigneten Filter (24),
einem Versorgungssystem (36), mittels dem Chemikalien zum Markieren der zu detektierenden Mikropartikel (32, 76, 78) mittels der Sonden (12, 80) durch das oder zu dem Filter (24) leitbar sind, und mittels dem Chemikalien zum Regenerieren des Filters mit Reinigen bzw. Konditionieren des Filters und des Fluidiksystems durch das oder zu dem Filter (24) leitbar sind, und
einem Detektionssystem (50, 52, 54) zum Erfassen (12, 80) der Sonden durch Erfassung von Strahlung (56),
wobei das Versorgungssystem (36) wenigstens ein Motorventil (A) aufweist, mit dem wahlweise einer von mehreren Versorgungs-Zuläufen (A2-A5, A7-A9) mit einem zu dem Filter (24) hin führenden Versorgungssystemablauf (A1) verbunden werden kann, wobei zwischen Filter (24) und Versorgungsablauf (A1) wenigstens eine Pumpe (34, 35) angeordnet ist.

2. Detektionsvorrichtung (20) nach Anspruch 1,
**dadurch gekennzeichnet,**
**dass** das Versorgungssystem (36) wenigstens eine Pumpe (34, 35) aufweist, sowie ein Ventilsystem (A), das an Quellen (A2 - A5, A7 - A9) für verschiedene Chemikalien für die Sondenmarkierung angeschlossen ist, um die jeweils für einzelne Markierungsschritte notwendige(n) Chemikalie(n) zu dem Filter (24) zu leiten, wobei das Ventilsystem wenigstens ein Motorventil (A) aufweist, mit dem wahlweise einer von mehreren Versorgungssystem-Zuläufen (A2 - A5, A7 - A9) mit einem zu dem Filter (24) hin führenden Versorgungssystemablauf (A1) verbunden werden kann.

3. Detektionsvorrichtung (20) nach einem der voranstehenden Ansprüche,
**dadurch gekennzeichnet,**
**dass** das Versorgungssystem (36) Teil eines Fluidiksystems (28) ist, mittels dem auch das zu messende Fluid (30) zu dem Filter (24) leitbar ist, wobei das Filter (24) in einer Detektionskammer (22) untergebracht ist, die an das Versorgungssystem (36) und/oder das Fluidiksystem (28) angeschlossen ist.

4. Detektionsvorrichtung (20) nach einem der voranstehenden Ansprüche,
**dadurch gekennzeichnet,**
**dass** das Detektionssystem ein Strahlungsmessgerät (50) zum Messen der von den Sonden (12, 80) stammenden Strahlung (56) und eine Auswerteeinheit (70) aufweist, die aufgrund der Messung durch das Strahlungsmessgerät (50) die Konzentration und/oder die Anzahl der Mikropartikel (32, 76, 78) bestimmt, wobei das Strahlungsmessgerät (50) einen ortsauflösenden, insbesondere einen wenigstens zweidimensional ortsauflösenden, Detektor (68) aufweist, der Strahlung (56) und deren Ursprungsposition auf dem Filter (24) erfassen kann.

5. Detektionsvorrichtung (20) nach Anspruch 4,
**dadurch gekennzeichnet,**
**dass** die Auswerteeinheit (70) aus der mit dem Photomultiplier (62) gemessenen Lichtintensität die Konzentration der Mikropartikel (32, 76, 78) bestimmt, wobei wenigstens ein optisches Filterelement (58) vorgesehen ist, das ausschließlich das von den Sonden (12, 80) ausgesandte Fluoreszenzlicht (56) zu einem Lichtdetektor (54) durchlässt.

6. Detektionsvorrichtung (20) nach einem der voranstehenden Ansprüche,
**dadurch gekennzeichnet,**
**dass** eine Steuerung zum vollautomatischen Steuern der Detektionsvorrichtung (20) vorgesehen ist.

7. Detektionsvorrichtung (20) nach einem der voranstehenden Ansprüche,
**dadurch gekennzeichnet,**
**dass** das Versorgungssystem (36) zur Lieferung von Chemikalien in der Weise ausgebildet und gesteuert ist, dass sich am Filter (24) befindliche lebende Bakterien (32) durch in-situ-Hybridisierung markiert werden, wobei sich die als DNA-Sonde (12) ausgebildete Sonde jeweils an die mRNA der zu detektierenden Bakterien (32) anlagert, wobei das Versorgungssystem (36) an eine Quelle angeschlossen ist, die mittels Strahlung (56) detektierbare DNA-Sonden (12) anliefert, die aufgrund einer Basensequenz derart ausgebildet sind, dass sie zu mehreren Bakterienarten einer Bakteriengruppe passt.

8. Detektionsvorrichtung (20) nach einem der voranstehenden Ansprüche,
**dadurch gekennzeichnet,**
**dass** ein Sammelsystem vorgesehen ist, in dem Mikropartikel (32) aus einem gasförmigen zu messenden Medium in einer Flüssigkeit gesammelt werden, wobei ein Flüssigkeitsablauf des Sammelsystems über das Filter (24) leitbar ist.

9. Detektionsvorrichtung (20) nach einem der voranstehenden Ansprüche, **gekennzeichnet durch** ein Zusatzmodul (72), mittels welchem eine PCR-Detektion von Mikropartikeln durchführbar ist.

10. Detektionsverfahren (20) zum Detektieren von biologischen Mikropartikeln, insbesondere von lebenden Bakterien (32), Viren (76) und/oder biologischen Toxinen (78)
**gekennzeichnet durch** die Schritte:
a) Leiten eines die biologischen Mikropartikel (32, 76, 78) enthaltenden Fluids (30) **durch** ein zum Ausfiltern der biologischen Mikropartikel (32, 76, 78) geeignetes Filter (24), wobei in Schritt a) eine zu untersuchende Flüssigkeitsprobe (30) mittels eines Fluidiksystems (28), das wenigstens eine Pumpe (34, 35) und wenigstens ein Ventil (A, B, C, D) zur Steuerung enthält, über das Filter (24) gepumpt wird,
b) Leiten von Chemikalien **durch** das, über das, oder zu dem Filter (24), um die daran anhaftenden biologischen Mikropartikel (32, 76, 78) mittels spezifischer Sonden (12, 80), die mittels Strahlung (56) erfassbar sind, zu markieren, wobei in Schritt b) auf dem Filter (24) befindliche Bakterien (32) einer in-situ-Hybridisierung unterzogen werden und wobei mit einem Fluoreszenzfarbstoff (11) versehene DNA-Sonden (12) verwendet werden,
c) Detektieren der mit den Sonden (12, 80) korrelierten Strahlung (56),
wobei das Fluid (30) und die Chemikalien über Versorgungssystem-Zuläufe (A2-A5, A7-A9) zu einem Motorventil (A) und dann über einen Versorgungssystemablauf (A1) zu der wenigstens einen Pumpe (34, 35) geleitet werden.

11. Detektionsverfahren nach Anspruch 10,
**dadurch gekennzeichnet,**
**dass** die biologischen Mikropartikel (32, 76, 78) aus der Luft oder einem anderen gasförmigen Medium in einer Flüssigkeit gesammelt werden und mit der Flüssigkeit über das Filter (24) geleitet werden.

12. Detektionsverfahren nach Anspruch 10 oder 11,
**gekennzeichnet durch** den Schritt:
d) Durchführen eines PCR-Verfahrens, wobei eine in-situ-PCR-Methode zur nachträglichen Detektion von Viren durchgeführt wird und wobei nach dem Verfahren der real-time-PCR während einer Amplifikation Reporter-Moleküle freigesetzt werden, die aufgrund ihrer Fluoreszenz mittels eines Lichtdetektors, insbesondere eines Photomultipliers, detektiert werden.

13. Detektionsverfahren nach Anspruch 12,
**dadurch gekennzeichnet,**
**dass** in Schritt b) eine in-situ-Hybrisierung in der Weise durchgeführt wird, dass nach dem Fixieren der Bakterien (32) DNA-Sonden (12) zugegeben werden, anschließend nichtgebundene DNA-Sonden weggespült werden, gebundene DNA-Sonden von der mRNA (14) gelöst und durch PCR amplifiziert werden.

14. Detektionsverfahren nach einem der Ansprüche 10 bis 13,
**dadurch gekennzeichnet,**
**dass** in Schritt a) die Probe durch ein mikromechanisches Filterelement (26) und/oder durch eine Nitrocellulosemembran (74) geleitet wird, die als das oder anstelle des oder zusätzlich zu dem mikromechanische(n) Filter(s) verwendet wird, wobei in Schritt b) noch freie Stellen der Nitrocellulosemembran (74) mit Proteinen (82) aufgefüllt werden und die biologischen Mikropartikel (32, 76, 78), die in der Membran (74) anhaften, mittels spezifischer Proteine, vorzugsweise Antikörper (80), oder markierter Sonden (12), die durch Strahlung (56) detektierbar sind, markiert werden.

15. Verwendung des Detektionsverfahrens nach einem der Ansprüche 10 bis 14 als Ersatz für eine Kultivierungsmethode zur Bestimmung der Lebendzellzahl in einer zu messenden Probe.

## Claims

1. A detecting device (20) for the detection of biological micro particles (32, 76, 78) that can be marked by means of probes (12, 80) that are detectable by means of radiation (56), wherein radiation-emitting nucleic acid probes (12) or radiation-emitting proteins are being used as probes (80), comprising:
a filter (24) suitable to filter out the micro particles (32, 76, 78) to be detected from a fluid (30) to be measured,
a supply system (36) by means of which chemicals for marking, by means of the probes (12, 80), the micro particles (32, 76, 78) to be detected can be conducted through or to the filter (24), and by means of which chemicals for regenerating the filter, including the cleaning or conditioning of the filter and the fluidic system, can be conducted through or to the filter (24), and
a detection system (50, 52, 54) for detecting (12, 80) the probes by detecting radiation (56),
wherein the supply system (36) has at least one motor valve (A) with which one of several supply inlets (A2-A5, A7-A9) can be selectively connected to a supply system outlet (A1) leading towards the filter (24), with at least one pump (34, 35) being disposed between the filter (24) and the supply outlet (A1).

2. The detection device (20) according to claim 1,
**characterised in that**
the supply system (36) includes at least one pump (34, 35) and a valve system (A) that is connected to sources (A2-A5, A7-A9) for various chemicals for marking probes in order to conduct the chemical(s) respectively required for individual marking steps to the filter (24), the valve system having at least one motor valve (A) with which one of several supply system inlets (A2-A5, A7-A9) can be selectively connected to a supply system outlet (A1) leading towards the filter (24).

3. The detecting device (20) according to any one of the preceding claims,
**characterised in that**
the supply system (36) is a part of a fluidic system (28) by means of which the fluid (30) to be measured can also be conducted to the filter (24), the filter (24) being accommodated in a detection chamber (22) that is connected to the supply system (36) and/or the fluidic system (28).

4. The detecting device (20) according to any one of the preceding claims,
**characterised in that**
the detection system comprises a radiation measuring device (50) for measuring the radiation (56) originating from the probes (12, 80) and an evaluation unit (70) that determines the concentration and/or the number of the micro particles (32, 76, 78) based on the measurement by the radiation measuring device (50), the radiation measuring device (50) having spatially-resolving, in particular an at least two-dimensionally spatially-resolving, detector (68) capable of detecting radiation (56) and its position of origin on the filter (24).

5. The detection device (20) according to claim 4,
**characterised in that**
the evaluation unit (70) determines the concentration of the micro particles (32, 76, 78) from the light intensity measured by the photo multiplier (62), with at least one optical filtering element (58) being provided that allows only the fluorescence light (56) emitted by the probes (12, 80) to pass through to a light detector (54).

6. The detecting device (20) according to any one of the preceding claims,
**characterised in that**
a control unit for controlling the detection device (20) fully automatically is provided.

7. The detecting device (20) according to any one of the preceding claims,
**characterised in that**
the supply system (36) for delivering chemicals is configured and controlled in such a manner that living bacteria (32) located on the filter (24) are marked by in situ hybridization, wherein the probe, which is configured as a DNA probe (12), respectively attaches to the mRNA of the bacteria (32) to be detected, the supply system (36) being connected to a source that delivers the DNA probes (12) that are detectable by radiation (56) and, due a base sequence, are configured in such a way that they match several bacterial species of a bacterial group.

8. The detecting device (20) according to any one of the preceding claims,
**characterised in that**
a collecting system is provided, in which micro particles (32) from a gaseous medium to be measured are collected in a liquid, wherein a liquid outlet of the collecting system can be conducted via the filter (24).

9. The detecting device (20) according to any one of the preceding claims, **characterised by** an additional module (72) by means of which a PCR detection of micro particles can be carried out.

10. A detection method (20) for detecting biological micro particles, in particular living bacteria (32), viruses (76), and/or biological toxins (78),
**characterised by** the steps:
a) conducting a fluid (30) containing the biological micro particles (32, 76, 78) through a filter (24) suitable for filtering out the biological micro particles (32, 76, 78), wherein, in step a), a liquid sample to be examined (30) is pumped via the filter (24) by means of a fluidic system (28) comprising at least one pump (34, 35) and at least one valve (A, B, C, D) for controlling,
b) conducting chemicals through, via or towards the filter (24) in order to mark the biological micro particles (32, 76, 78) adhering thereto by means of specific probes (12, 80) that can be detected by means of radiation (56), wherein, in step b), bacteria (32) located on the filter (24) are subjected to an in situ hybridization and wherein DNA probes (12) provided with a fluorescent dye (11) are used,
c) detecting the radiation (56) correlated with the probes (12, 80),
wherein the fluid (30) and the chemicals are conducted via supply system inlets (A2-A5, A7-A9) to a motor valve (A) and then via a supply system outlet (A1) to the at least one pump (34, 35).

11. The detection method according to claim 10,
**characterised in that**
the biological micro particles (32, 76, 78) are collected from the air or another gaseous media in a liquid and are conducted together with the liquid via the filter (24).

12. The detection method according to claim 10 or 11,
**characterised by** the step:
d) carrying out a PCR process, wherein an in situ PCR process is carried out for the subsequent detection of viruses and wherein, in accordance with the real-time-PCR process, reporter molecules are released during an amplification that are detected by a light detector, in particular a photo multiplier, due to their fluorescence.

13. The detection method according to claim 12,
**characterised in that**
in step b), an in situ hybridization is carried out in such a way that after fixating the bacteria (32), DNA probes (12) are added, non-bound DNA probes are then washed away, and bound DNA probes are disengaged from the mRNA (14) and amplified by PCR.

14. The detection method according to any one of the claims 10 to 13,
**characterised in that**
in step a), the sample is conducted through a micromechanical filtering element (26) and/or through a nitrocellulose membrane (74) that is used as, instead of, or in addition to, the micromechanical filtering element, wherein, in step b), areas of the nitrocellulose membrane (74) that are still unoccupied are filled up with proteins (82) and the biological micro particles (32, 76, 78) adhering in the membrane (74) are marked by means of specific proteins, preferably by antibodies (80), or by marked probes (12) that can be detected by radiation (56).

15. Use of a detection method according to any one of the claims 10 to 14 as a substitute for a cultivation method for determining the number of living cells in a sample to be measured.

## Revendications

1. Dispositif de détection (20) pour la détection de microparticules biologiques (32, 76, 78) qui sont susceptibles d'être marquées par des sondes (12, 80) et qui peuvent être décelées au moyen d'un rayonnement (56), dans lequel on utilise des sondes d'acides nucléiques (12) émettant un rayonnement ou on utilise à titre de sondes (80) des protéines émettant un rayonnement, comprenant
un filtre (24) approprié pour filtrer les microparticules à détecter (32, 76, 78) hors d'un fluide à mesurer (30),
un système d'alimentation (36) au moyen duquel des produits chimiques destinés à marquer les microparticules à détecter (32, 76, 78) au moyen des sondes (12, 80) peuvent être passés à travers le filtre (24) ou vers celui-ci, et au moyen duquel des produits chimiques destinés à régénérer le filtre lors du nettoyage ou du conditionnement du filtre et du système fluidique peuvent être passés à travers le filtre (24) ou vers celui-ci, et
un système de détection (50, 52, 54) pour déceler (12, 80) les sondes en décelant le rayonnement (56),
dans lequel le système d'alimentation (36) comprend au moins une vanne motorisée (A) au moyen de laquelle on peut relier au choix une arrivée d'alimentation parmi plusieurs (A2-A5, A7-A9) avec une sortie du système d'alimentation (A1 qui mène au filtre (24), et dans lequel au moins une pompe (34, 35) est agencée entre le filtre (24) et la sortie d'alimentation (A1).

2. Dispositif de détection (20) selon la revendication 1,
**caractérisé en ce que** le système d'alimentation (36) comprend au moins une pompe (34, 35), ainsi qu'un système de vannes (A) qui est branché à des sources (A2-A5, A7-A9) pour différents produits chimiques destinés au marquage par les sondes, afin d'amener au filtre (24) le ou les produits chimiques respectifs nécessaires pour des opérations de marquage individuelles, dans lequel le système de vannes comprend au moins une vanne motorisée (A) au moyen de laquelle on peut relier au choix une arrivée d'alimentation parmi plusieurs (A2-A5, A7-A9) avec une sortie du système d'alimentation (A1) qui mène au filtre (24).

3. Dispositif de détection (20) selon l'une des revendications précédentes,
**caractérisé en ce que** le système d'alimentation (36) et une partie d'un système fluidique (28) au moyen duquel le fluide à mesurer (30) peut également être amené au filtre (24), dans lequel le filtre (24) est logé dans une chambre de détection (22) qui est branché au système d'alimentation (36) et/ou au système fluidique (28).

4. Dispositif de détection (20) selon l'une des revendications précédentes,
**caractérisé en ce que** le système de détection comprend un appareil de mesure de rayonnement (50) pour mesurer le rayonnement (56) provenant des sondes (12, 80) et une unité d'évaluation (70) qui détermine, au moyen de la mesure par l'appareil de mesure de rayonnement (50), la concentration et/ou le nombre des microparticules (32, 76, 78), dans lequel l'appareil de mesure de rayonnement (50) comprend un détecteur à résolution locale, en particulier un détecteur (68) à résolution locale au moins bidimensionnel, qui est capable de déceler le rayonnement (56) et la position de son origine sur le filtre (24).

5. Dispositif de détection (20) selon la revendication 4,
**caractérisé en ce que** l'unité d'évaluation (70) détermine, à partir de l'intensité de lumière mesurée avec le photomultiplicateur (62), la concentration des microparticules (32, 76, 78), et il est prévu au moins un élément filtre optique (58) qui laisse exclusivement passer la lumière fluorescente (56) émise depuis des sondes (12, 80) vers un détecteur de lumière (54).

6. Dispositif de détection (20) selon l'une des revendications précédentes,
**caractérisé en ce qu'**il est prévu une commande pour la commande entièrement automatique du dispositif de détection (20).

7. Dispositif de détection (20) selon l'une des revendications précédentes,
**caractérisé en ce que**, pour la fourniture de produits chimiques, le système d'alimentation (36) est réalisé et commandé de telle façon que des bactéries vivantes (32) qui se trouvent sur le filtre (24) sont marquées par hybridation in situ, de sorte que la sonde réalisée sous forme de sonde ADN (12) vient se déposer respectivement sur le ARNm des bactéries à détecter (32), et dans lequel le système d'alimentation (36) est branché à une source qui fournit des sondes ADN (12) susceptibles d'être détectées au moyen d'un rayonnement (56), qui sont réalisées, en raison d'une séquence de base, de telle façon qu'elles s'adaptent à plusieurs sortes de bactéries d'un groupe de bactéries.

8. Dispositif de détection (20) selon l'une des revendications précédentes,
**caractérisé en ce qu'**il est prévu un système de collecte dans lequel des microparticules (32) provenant d'un milieu gazeux à mesurer sont collectées dans un liquide, et une sortie de liquide du système de collecte peut être passée par le filtre (24).

9. Dispositif de détection (20) selon l'une des revendications précédentes,
**caractérisé par** un module additionnel (72), au moyen duquel une détection dite PCR ("Polymerase Chain Reaction" ou "réaction à chaîne polymérase") des microparticules peut être exécutée.

10. Procédé de détection (20) pour la détection de microparticules biologiques, en particulier de bactéries vivantes (32), de virus (76) et/ou de toxines biologiques (78),
**caractérisé par** les étapes consistant à :
a) faire passer un fluide (30) contenant les microparticules biologiques (32, 76, 78) à travers un filtre (24) approprié pour filtrer les microparticules biologiques (32, 76, 78), de sorte que l'on pompe un échantillon de liquide à analyser (30) dans l'étape a) via le filtre (24), au moyen d'un système fluidique (28) qui contient au moins une pompe (34, 35) et au moins une vanne (A, B, C, D),
b) faire passer des produits chimiques à travers le filtre (24), via celui-ci ou vers celui-ci, afin de marquer les microparticules biologiques (32, 76, 78) qui adhèrent sur le filtre au moyen de sondes spécifiques (12, 80) qui peuvent être décelées au moyen d'un rayonnement (56), de sorte que l'on soumet dans l'étape b) les bactéries (32) qui se trouvent sur le filtre (24) à une hybridation in situ, et l'on utilise des sondes ADN (12) dotées d'un colorant à fluorescence (11),
c) détecter le rayonnement (56) en corrélation avec les sondes (12, 80), dans lequel le fluide (30) et les produits chimiques sont amenés via des arrivées d'alimentation (A2-A5, A7-A9) vers une vanne motorisée (A) et ensuite via une sortie du système d'alimentation (A1) vers ladite au moins une pompe (34, 35).

11. Procédé de détection selon la revendication 10,
**caractérisé en ce que** les microparticules biologiques (32, 76, 78) sont collectées depuis l'air ou depuis un autre milieu gazeux dans un liquide et sont amenées avec le liquide via le filtre (24).

12. Procédé de détection selon la revendication 10 ou 11,
**caractérisé par** l'étape consistant à :
d) exécuter une procédure PCR, dans laquelle on exécute un procédé PCR in situ pour la détection a posteriori de virus, et dans lequel après la procédure de PCR en temps réel des molécules traceuses sont dégagées pendant une amplification, lesquelles sont détectées en raison de leur fluorescence au moyen d'un détecteur de lumière, en particulier d'un photomultiplicateur.

13. Procédé de détection selon la revendication 12,
**caractérisé en ce que** l'on exécute dans l'étape b) une hybridation in situ, de telle manière qu'après avoir fixé les bactéries (32) on ajoute des sondes ADN (12), on rince ensuite les sondes ADN qui ne sont pas liées, on détache les sondes ADN liées depuis le ARNm (14) et on les amplifie par PCR.

14. Procédé de détection selon l'une des revendications 10 à 13, **caractérisé en ce que** dans l'étape a) l'échantillon est amené à travers un élément filtrant micromécanique (26) et/ou à travers une membrane de nitrocellulose (74) qui est utilisée à titre de filtre micromécanique ou à la place de celui-ci ou encore en supplément de celui-ci, de sorte que dans l'étape b) des emplacements encore libres de la membrane de nitrocellulose (74) sont remplies avec des protéines (82) et les microparticules biologiques (32, 76, 78) qui adhèrent dans la membrane (74) sont marquées au moyen de protéines spécifiques, de préférence des anticorps (80) ou de sondes marquées (12) qui peuvent être détectées par rayonnement (56).

15. Utilisation du procédé de détection selon l'une des revendications 10 à 14 à titre de remplacement pour une méthode de culture en vue de la détermination du nombre de cellules vivantes dans un échantillon à mesurer.
